# EUROPÄISCHE PATENTANMELDUNG

(11) **EP 1 795 893 A1**
(43) Veröffentlichungstag der Anmeldung: **13.06.2007**
(21) Anmeldenummer: 06123717.8
(22) Anmeldetag: 09.11.2006
(51) Int. Cl.: G01N 33/34, G01N 3/08

(54) **Verfahren zur Bestimmung einer Dehnungseigenschaft einer Bahn**

(30) Priorität: 06.12.2005 DE 102005058142
(71) Anmelder: Voith Patent GmbH, 89522 Heidenheim (DE)
(72) Erfinder: Muench, Rudolf, 89551 Koenigsbronn (DE); Schoelzke, Volker, 47798 Krefeld (DE)

(57) **Zusammenfassung**

Verfahren zur Bestimmung zumindest einer Dehnungseigenschaft einer in einer Maschine (1;10) zur Herstellung einer Faserstoffbahn laufenden Bahn mit folgenden Schritten:
- Führen der Bahn über zumindest zwei aufeinander folgende angetriebene Walzen (4,6;12,13,14), wobei die Bahn zwischen aufeinander folgenden angetriebenen Walzen (4,6;12,13,14) jeweils zumindest abschnittweise ungestützt ist,
- Ermittlung der Zugkraft in der Bahn zwischen den aufeinander folgenden Walzen (4,6;12,13,14),
- Ermittlung der Differenzgeschwindigkeit der aufeinander folgenden Walzen (4,6;12,13,14) zueinander,
- Bestimmung der zumindest einen Dehnungseigenschaft der zwischen den angetriebenen Walzen (4,6;12,13,14) geführten Bahn unter Berücksichtigung der zumindest einen Differenzgeschwindigkeit und der zumindest einen Zugkraft.

## Beschreibung

Die Erfindung betrifft ein Verfahren zur Bestimmung zumindest einer Dehnungseigenschaft einer in einer Maschine zur Herstellung einer Faserstoffbahn laufenden Bahn. Bei der Bahn kann es sich um eine Faserstoffbahn wie auch um eine Papiermaschinenbespannung handeln.

Mit den aus dem Stand der Technik bekannten Verfahren können Dehnungseigenschaften wie beispielsweise Elastizität oder Festigkeitseigenschaften, wie beispielsweise Spaltbarkeit, Bruchdehnung oder Tensile, bisher entweder nur offline, das heißt nach Probenentnahme im Labor oder aber mittels speziellen Sensoren, wie beispielsweise Ultraschallsensoren ermittelt werden.

Die Offline-Messung hat darüber hinaus den Nachteil, dass eine unmittelbare Reaktion während der laufenden Produktion auf Schwankungen der Bahnelastizität und Bahnfestigkeit außerhalb des Normbereichs nicht möglich ist, was aufgrund der dadurch bedingten Zeitverzögerung zu hoher Ausschussproduktion führen kann.

Beides, die Labormessung und die Messung mit speziellen Sensoren, ist hierbei sehr kostenaufwändig.

Auch die Messung von Dehnungseigenschaften von Maschinenbespannungen, dies kann beispielsweise hilfreich sein, um die noch bevorstehende Lebensdauer der Bespannung vorherzusagen, erfordert oftmals spezielle Vorrichtungen mit denen über die Siebspannung auf diese geschlossen werden kann.

Es ist die Aufgabe der vorliegenden Erfindung, ein Verfahren der eingangs genannten Art vorzuschlagen, bei dem die beschriebenen Nachteile nicht mehr auftreten.

Die Aufgabe wird erfindungsgemäß durch die Merkmale des Patentanspruchs 1 gelöst.

Erfindungsgemäß wird ein Verfahren zur Bestimmung zumindest einer Dehnungseigenschaft einer in einer Maschine zur Herstellung einer Faserstoffbahn laufenden Bahn mit folgenden Schritten vorgeschlagen:

Es wird die Bahn über zumindest zwei aufeinander folgende angetriebene Walzen geführt, wobei die Bahn zwischen aufeinander folgenden angetriebenen Walzen jeweils zumindest abschnittweise ungestützt ist. Ferner ist vorgesehen, dass die Zugkraft der Bahn zwischen den aufeinander folgenden Walzen wie auch die Differenzgeschwindigkeit der aufeinander folgenden Walzen zueinander ermittelt wird. Des Weiteren wird die zumindest eine Dehnungseigenschaft der zwischen den angetriebenen Walzen geführten Bahn unter Berücksichtigung der zumindest einen Differenzgeschwindigkeit und der zumindest einen Zugspannung bestimmt.

Das erfindungsgemäße Verfahren beruht auf der Idee, Dehnungseigenschaften der Bahn, das heißt der Faserstoffbahn oder der Maschinenbespannung, auf der Basis von Größen zu ermitteln, die bei Maschinen zur Herstellung einer Faserstoffbahn bereits als Regelgrößen beziehungsweise Parameter für die Herstellung der Faserstoffbahn bestimmt und geregelt werden. Somit kann mit dem erfindungsgemäßen Verfahren auf bereits in heutigen Maschinen zur Herstellung einer Faserstoffbahn vorhandene Systeme zurückgegriffen werden um Dehnungseigenschaften der Bahn zu bestimmen.

Da die Differenzgeschwindigkeit zwischen den angetriebenen Walzen ein Maß für die Dehnung der Bahn ist, kann mit dem erfindungsgemäßen Verfahren nach dem Hook'schen Gesetz bei bekannter Zugkraft der Elastizitätsmodul berechnet werden, weshalb es sich vorzugsweise bei der Dehnungseigenschaft der Bahn um deren E-Modul dreht. Mit dem erfindungsgemäßen Verfahren ist somit eine direkte Bestimmung des Elastizitätsmoduls möglich.

Da sowohl die Zugkraft in der Bahn wie auch die Differenzgeschwindigkeit in der Regel kontinuierlich während des Herstellungsprozesses ermittelt werden, kann die Dehnungseigenschaft des weiteren während des gesamten Prozesses laufend ermittelt werden und somit unerwünschte Änderungen während der Produktion korrigiert werden.

Vorteilhafte Ausführungsformen und Weiterbildungen der Erfindung sind in den Unteransprüchen angegeben.

Vorzugsweise werden zur Bestimmung der zumindest einen Dehnungseigenschaft, die zeitliche Änderung der Zugkraft und/oder die zeitliche Änderung der Differenzgeschwindigkeit berücksichtigt werden.

Maschinen zur Herstellung einer Faserstoffbahn sind in der Regel geschwindigkeitsgesteuert, das heißt zwischen aufeinander folgenden angetriebenen Walzen wird eine konstante Differenzgeschwindigkeit eingestellt und es wird nur die Zugkraft auf die Bahn derart geregelt, dass die vorgegebene Differenzgeschwindigkeit konstant bleibt. In diesem Fall wird demzufolge vorzugsweise zur Bestimmung einer in Bahnlaufrichtung unterschiedlichen Dehnungseigenschaft der Bahn, die zeitliche Änderung der jeweiligen Zugkraft ermittelt und die Differenzgeschwindigkeit zwischen den aufeinander folgenden Walzen zeitlich konstant gehalten.

Anders als oben angegeben wird beispielsweise im Bereich das Aufwickelns der Bahnzug der aufwickelnden Walze konstant gehalten und die Differenzgeschwindigkeit zwischen der aufwickelnden Walze und der vorher angetriebenen Walze zur Einstellung des konstanten Bahnzugs entsprechend geregelt. In diesem Fall wird vorzugsweise zur Bestimmung einer in Bahnlaufrichtung unterschiedlichen Dehnungseigenschaft der Bahn, die zeitliche Änderung der jeweiligen Differenzgeschwindigkeit ermittelt und die Zugkraft der Bahn zwischen den jeweils aufeinander folgenden Walzen zeitlich konstant gehalten wird.

Wie bereits angedeutet, soll der erfindungsgemäße Gedanke nicht nur auf eine Faserstoffbahn, wie insbesondere eine Papier-, Karton- oder Tissuebahn beschränkt bleiben, sondern soll vielmehr auch eine Bespannung, insbesondere ein Formiersieb, ein Pressfilz, ein Trockensieb oder ein glattes Transferband umfassen.

Bei heutigen Maschinen zur Herstellung einer Faserstoffbahn werden die Antriebsmomente der angetriebenen Walzen kontinuierlich geregelt und deren Ist-Zustände erfasst. Da über die Antriebsmomente auch die Zugkraft in der Bahn beeinflusst werden kann, sieht eine bevorzugte Weiterbildung der Erfindung vor, dass die Zugkraft der Bahn unter Berücksichtigung der jeweiligen Antriebsdrehmomente der Walzenantriebe ermittelt wird, wobei die Antriebsdrehmomente beispielsweise aus der Kenntnis der Werte der Antriebsparameter der Walzenantriebe, insbesondere der elektrischen Spannung, dem elektrischen Strom oder der Leistungsaufnahme, ermittelt werden.

Vorzugsweise gehen in die Ermittlung der Zugkraft des weiteren Kenndaten der Getriebe der Walzenantriebe und/oder Kenndaten der Walzen, wie insbesondere der Reibwiderstand der Lagerung der Walze, der Walzendurchmesser und dergleichen mit ein. Somit lässt sich auf Basis der Werte der Antriebsparameter sehr zuverlässig die Zugkraft der Bahn bestimmen.

Oftmals tritt ein Schlupf zwischen den angetriebenen Walzen und der auf diesen geführten Bahn auf. Um die Genauigkeit des Wertes der ermittelten Dehnungseigenschaft zu erhöhen, sieht eine weitere bevorzugte Ausgestaltung der Erfindung vor, dass für die Bestimmung der zumindest einen Dehnungseigenschaft Reibwerte zwischen der Bahn und den angetriebenen Walzen berücksichtigt werden.

In diesem Fall ist es sogar möglich, dass ein Absolutwert der zumindest einen Dehnungseigenschaft bestimmt werden kann.

Selbstverständlich können zwischen den angetriebenen Walzen eine oder mehrere nicht angetriebene Walzen angeordnet sind, über welche die Bahn geführt wird. Bei den nicht angetrieben Walzen kann es sich beispielsweise um Leitwalzen oder Umlenkwalzen handeln.

Auch kann zwischen den angetriebenen Walzen ein bekannter Blaskasten mit einer gekrümmten Blasfläche angeordnet sein, über welchen die Bahn geführt wird. Zwischen der gekrümmten Blasfläche des Blaskastens und der Bahn kann somit ein Luftpolster erzeugt werden, welches die zwischen den angetriebenen Walzen geführte Bahn dehnt. Aus der ermittelbaren Dehnung der Bahn, dem Krümmungsradius des Blaskastens und dem Druck zur Erzeugung des Luftpolsters kann wiederum die Zugkraft in der Bahn ermittelt werden. Die gekrümmte Blasfläche des Blaskastens kann aus einem Leistenbelag oder einem gelochten Belag bestehen.

Es sind unterschiedliche Konfigurationen denkbar, wie die zumindest eine Dehnungseigenschaft bestimmt werden kann.

So kann diese beispielsweise zwischen zwei aufeinander folgenden angetriebenen Walzen bestimmt werden. In diesem Fall sind die zwei aufeinander folgenden angetriebenen Walzen vorzugsweise die beiden letzten Walzen der Maschine zur Herstellung einer Faserstoffbahn, wobei die letzte Walze die Walze sein kann, auf welche die Faserstoffbahn aufgewickelt wird. Mit dieser Konfiguration ist eine besonders einfache Bestimmung der zumindest einen Dehnungseigenschaft möglich, da das Antriebsmoment des letzten Antriebs durch keine weitere Zugkraft beeinflusst wird, das heißt es kann aus den Antriebsmomenten der letzten beiden Antriebe einfach auf den Bahnzug geschlossen werden. Aus den Bahnzugänderungen und der Differenzgeschwindigkeit kann dann die Änderung der Elastizitätskonstante bestimmt werden.

In diesem Fall entspricht die örtliche Auflösung bzgl. der Dehnungseigenschaft der Bahn dem räumlichen Abstand der beiden angetriebenen Walzen.

Eine andere Konfiguration sieht vor, dass die zumindest eine Dehnungseigenschaft zwischen drei aufeinander folgenden angetriebenen Walzen bestimmt wird. In dieser Konfiguration ist es möglich, die Dehnungseigenschaft an beliebiger Position, das heißt auch vor dem Aufrollen, in der Maschine zu ermitteln, so dass die erhaltenen Ergebnisse sowohl zur Vorwärtsregelung als auch zur entsprechenden weiteren Beeinflussung der Faserstoffbahn in den nachfolgenden Prozessschritten herangezogen werden können.

Die Bestimmung der zumindest einen Dehnungseigenschaft zwischen drei aufeinander folgenden angetriebenen Walzen ist überall an den Positionen der Maschine zur Herstellung einer Faserstoffbahn sinnvoll, wo in Bahnlaufrichtung weitere angetriebene Walzen folgen.

Handelt es sich bei der Bahn um eine in einer geschlossenen Schleife umlaufenden Maschinenbespannung so kann mittels obiger Konfiguration eine Dehnungseigenschaft der Bespannung ermittelt werden.

In diesem Zusammenhang ist es denkbar, dass die zumindest eine Dehnungseigenschaft unter Berücksichtigung der zeitlichen Änderung des Antriebsdrehmoments der mittleren der drei angetriebenen Walzen bestimmt wird.

Änderungen der Dehnungseigenschaft der Bahn treten bei der Konfiguration mit drei aufeinander folgenden angetriebenen Walzen immer zuerst vor der mittleren der Walzen auf, das heißt zwischen erster und mittlerer Walze, bevor diese Änderung nach einer gewissen Transportzeit nach der mittleren Walze, das heißt zwischen mittlerer und letzter Walze wirksam werden.

Da das von der mittleren Walze beispielsweise zur Aufrechterhaltung einer vorgegebenen konstanten Differenzgeschwindigkeit aufgebrachte Antriebsmoment immer von den Eigenschaften der Bahn vor und nach der mittleren Walze beeinflusst wird, ist es in diesem Zusammenhang sinnvoll, wenn die zumindest eine Dehnungseigenschaft unter Berücksichtigung des Integrals des Antriebsdrehmoments der mittleren der drei angetriebenen Walzen, wobei über die Zeit integriert wird.

Hierbei wird vorzugsweise über die Zeit integriert, die ein Ort auf der Bahn benötigt, um von der ersten zur dritten angetriebenen Walze zu gelangen. In diesem Fall entspricht die örtliche Auflösung bzgl. der Dehnungseigenschaft der Bahn dem räumlichen Abstand zwischen erster und letzter der drei angetriebenen Walzen.

Vorzugsweise handelt es sich der Dehnungseigenschaft um die Elastizität der Bahn.

Nach einer weiteren bevorzugten Ausgestaltung der Erfindung ist es denkbar, dass aus der ermittelten Dehnungseigenschaft der Bahn, insbesondere Faserstoffbahn, zumindest eine Festigkeitseigenschaft der Bahn ermittelt wird. Hierbei wird beispielsweise der Ist-Wert zumindest einer die Festigkeitseigenschaft beeinflussenden Regelgröße im Herstellungsprozess der Bahn bestimmt und mittels eines Modells, welches eine Abhängigkeit der Festigkeitseigenschaft von der Dehnungseigenschaft und von der Regelgröße herstellt, die Festigkeitseigenschaft ermittelt. Anstelle der Regelgröße kann auch ein Parameter verwendet werden. Das Modell kann hierbei zusätzlich die Sorte der Bahn und/oder spezifische Eigenarten des verwendeten Herstellungsprozesses und/oder die Bauart der die Bahn herstellenden Maschine berücksichtigen. Des Weiteren kann das Modell im Labor gemessene Werte der zumindest einen Festigkeitseigenschaft berücksichtigen. Das Modell kann hierbei insbesondere einen mathematischen Algorithmus, wie beispielsweise PCA, PLSR, SOM, Fuzzy umfassen oder ein neuronales Netzwerk umfassen.

Bezüglich der konkreten Ausgestaltung eines Sensor-Systems, durch welches die oben ausgeführten Prozessschritte durchführt werden können, wird auf die europäischen Patentanmeldung mit der Veröffentlichungsnummer EP 1 517 207 A2 verwiesen, die diesbezüglich vollumfänglich in den Offenbarungsgehalt dieser Anmeldung mit aufgenommen wird.

Die zumindest eine Festigkeitseigenschaft der Faserstoffbahn umfasst vorzugsweise: die Spaltbarkeit, die Längs- und/oder Querbruchdehnbarkeit, den Tensile oder die Reißlänge der Faserstoffbahn.

Nachfolgend wird die Erfindung anhand von Ausführungsbeispielen durch die folgenden Zeichnungen weiter erläutert.

Es zeigen:
- Figur 1: einen Teilabschnitt einer Maschine zur Herstellung einer Faserstoffbahn, der zur Durchführung einer Ausführungsform des erfindungsgemäßen Verfahrens geeignet ist, in schematischer Darstellung;
- Figur 2: einen Teilabschnitt einer Maschine zur Herstellung einer Faserstoffbahn, der zur Durchführung einer zweiten Ausführungsform des erfindungsgemäßen Verfahrens geeignet ist, in schematischer Darstellung; und
- Figur 3: einen Teilabschnitt einer Maschine zur Herstellung einer Faserstoffbahn, der zur Durchführung einer dritten Ausführungsform des erfindungsgemäßen Verfahrens geeignet ist, in schematischer Darstellung.

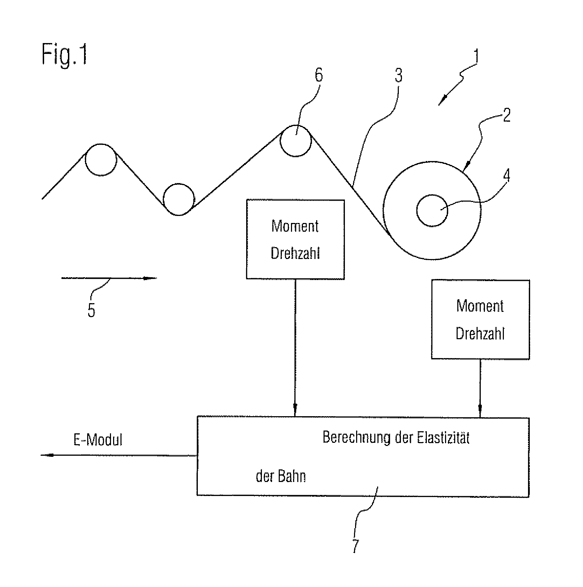

Die Figur 1 zeigt einen Teilabschnitt einer Maschine 1 zur Herstellung einer Faserstoffbahn 3 mit einer Aufwickelvorrichtung 2, bei welcher die Faserstoffbahn 3 auf eine angetriebene Walze 4 aufgewickelt wird. Die Walze 4 stellt die letzte Walze der Maschine 1 dar. In Bahnlaufrichtung 5 (Pfeil) vor der letzten Walze 4 ist eine weitere angetriebene Walze 6 angeordnet, welche beispielsweise durch die letzte Walze einer Trockenpartie der Maschine 1 gebildet werden kann. Die Faserstoffbahn 3 wird zwischen den beiden angetriebenen Walzen 4 und 6 ungestützt geführt.

Zur Bestimmung beispielsweise der Elastizität der Faserstoffbahn 3 vor dem Aufwickeln wird die Zugkraft in der Faserstoffbahn 3 zwischen den beiden aufeinander folgenden Walzen 6 und 4 auf einem vorbestimmten Wert zeitlich konstant gehalten, so dass die Antriebsmomente und daraus folgt die Leistungsaufnahme der beiden angetriebenen Walzen 4 und 6 keiner zeitlichen Veränderung unterworfen sind. Das weiteren wird mittels nicht dargestellter Sensoren die zeitliche Änderung der jeweiligen Differenzgeschwindigkeit, das heißt die Drehzahldifferenz der beiden Walzen 4 und 6 zueinander bestimmt. Da die Differenzgeschwindigkeit ein Maß für die Dehnung der Faserstoffbahn 3 ist, lässt sich daraus das Elastizitätsmodul das Abschnitts der Faserstoffbahn 3 bestimmen, der zwischen den beiden angetriebenen Walzen 4 und 6 geführt wird.

Dementsprechend werden Daten bzgl. der Drehzahl und dem Antriebsmoment von jeder der beiden angetriebenen Walzen 4 und 6 ausgelesen und einer Recheneinheit 7 zugeführt, welche daraus den E-Modul der Faserstoffbahn 3 berechnet.

Vorzugsweise werden des weiteren u.a. der Reibwerte zwischen der Faserstoffbahn 3 und den angetriebenen Walzen 4, 6 wie auch weitere Kenndaten der Walzen 4 und 6 und deren Getriebe, beispielsweise Walzendurchmesser mit berücksichtigt, so dass beispielsweise mittels eines entsprechenden Modells direkt aus der Umdrehungsdrehzahl der Walzenachsen auf die Differenzgeschwindigkeit geschlossen werden kann.

Durch die Verfolgung der zeitlichen Änderung der Differenzgeschwindigkeit kann somit eine Änderung der Elastizität der laufend hergestellten und auf der Wickeleinrichtung 2 aufgewickelten Faserstoffbahn 3 verfolgt werden. Somit lässt sich beispielsweise ein Abdriften des Faserstoffherstellungsprozesses, der sich in einer Änderung der Elastizität der Faserstoffbahn 3 bemerkbar macht, während dem laufenden Herstellungsprozess detektieren, damit darauf basierend, entsprechende Gegenmaßnahmen durch Beeinflussung entsprechender Regelparameter eingeleitetet werden können.

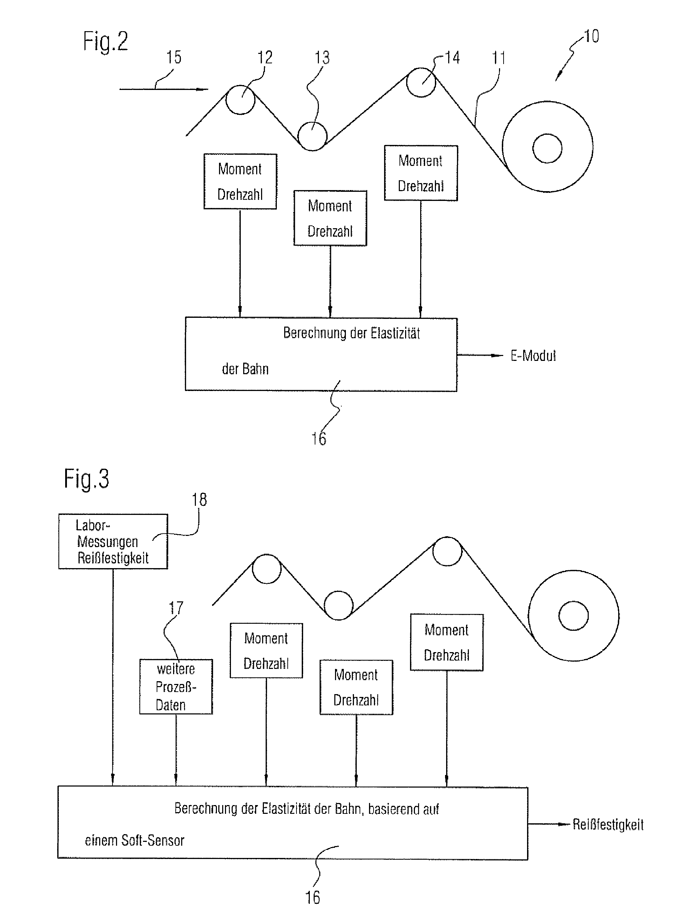

Die Figur 2 zeigt einen Teilabschnitt einer Maschine 10 zur Herstellung einer Faserstoffbahn 11. Bei dem Teilabschnitt kann es sich beispielsweise um einen Bereich in der Trockenpartie der Maschine 10 handeln.

Der Teilabschnitt weist drei aufeinander folgende angetriebene Walzen 12, 13 und 14 auf, über welche die Faserstoffbahn 11 in Bahnlaufrichtung 15 (Pfeil) geführt wird und zwischen denen die Faserstoffbahn 11 jeweils ungestützt geführt wird.

Im Bereich der Trockenpartie wird die Differenzgeschwindigkeit zwischen den aufeinander folgenden angetriebenen Walzen 12 und 13 sowie die Differenzgeschwindigkeit zwischen den aufeinander folgenden angetriebenen Walzen 13 und 14 jeweils auf einem festgelegten Wert konstant gehalten, wohingegen die Zugkraft auf die Faserstoffbahn 11 zur Aufrechterhaltung der jeweils vorgegebenen Differenzgeschwindigkeit entsprechend zwischen den Walzen 12 und 13 sowie zwischen den Walzen 13 und 14 geregelt wird.

Da im vorliegenden Beispiel die Elastizität der Faserstoffbahn 11 nicht am Ende der Maschine 10, sondern vorher bestimmt wird, werden demzufolge zu deren Ermittlung die zeitlich konstanten Differenzgeschwindigkeiten sowie die Zugkraft in der Faserstoffbahn 11 zwischen den aufeinander folgenden Walzen 12 und 13 sowie die Zugkraft in der Faserstoffbahn 11 zwischen den aufeinander folgenden Walzen 13 und 14 ermittelt. Die Werte werden einer Recheneinheit 16 zugeführt, welche daraus den E-Modul der Faserstoffbahn 11 berechnet.

Alternativ dazu kann das E-Modul aufgrund von folgenden Überlegungen bestimmt werden:

Eine Änderung der Elastizität der Faserstoffbahn 11 tritt immer zuerst vor der mittleren Walze 13 auf, bevor diese Änderung nach einer gewissen Transportzeit nach der mittleren Walze 13, das heißt zwischen mittlerer Walze 13 und letzter Walze 14 wirksam wird. Daher geht in das von der mittleren Walze 13 bereitgestellte Antriebsmoment immer die Elastizität der Faserstoffbahn 11 vor und nach der mittleren Walze 13 ein. Das heißt das Antriebsmoment der mittleren Walze kann als Maß für den Bahnzug vor und nach der mittleren Walze 13 betrachtet werden.

Befindet sich beispielsweise ein Abschnitt der Faserstoffbahn 11 mit einer geringeren Elastizität zwischen der ersten Walze 12 und der mittleren Walze 13, so muss die mittlere Walze 13 zur Bereitstellung der konstant vorgegebenen Differenzgeschwindigkeit ein kleineres Antriebsmoment als sonst aufbringen, da diese von der nachfolgenden Walze 14 gleich gezogen wird, diese selbst aber aufgrund der geringeren Elastizität des Bahnabschnitts weniger Zug aufbringen muss. Dies macht sich beispielsweise in einer geringeren Leistungsaufnahme der mittleren Walze 13 bemerkbar.

Befindet sich dann der Abschnitt mit der geringeren Elastizität aufgrund des Weitertransports der Faserstoffbahn 11 zwischen der mittleren Walze 13 und der nachfolgenden angetriebenen Walze 14, so muss die mittlere Walze 13 zur Bereitstellung der konstant vorgegebenen Differenzgeschwindigkeit ein höheres Antriebsmoment als sonst aufbringen, da diese von der nachfolgenden Walze 14 aufgrund der geringeren Elastizität der Faserstoffbahn 11 weniger gezogen wird, diese selbst aber aufgrund der wieder höheren Elastizität des Bahnabschnitts zwischen den ersten beiden Walzen 12 und 13 mehr Zug aufbringen muss.

Bildet man das Integral des Antriebsdrehmoments der mittleren angetriebenen Walze 13 über die Zeit, wobei über die Zeit integriert wird, die ein Ort auf der Faserstoffbahn 11 benötigt, um von der ersten Walze 12 zur dritten angetriebenen Walze 14 zu gelangen, so lässt sich aus dem Integral über das Antriebsdrehmoment bzw. über die Leistungsaufnahme das E-Modul der Faserstoffbahn 11 berechnen. Die örtliche Auflösung bzgl. der Elastizität der Faserstoffbahn 11 entspricht hierbei dem räumlichen Abstand zwischen erster Walze 12 und letzter angetriebener Walze 14.

Die Figur 3 zeigt eine weitere erfindungsgemäße Ausführungsform, welche der der Figur 2 entspricht, mit dem Zusatz, dass aus der ermittelten Elastizität der Faserstoffbahn 11 die Reißlänge der Faserstoffbahn 11 ermittelt wird. Hierzu werden die Reißlänge beeinflussende Regelgrößen auf Basis von Prozessdaten bestimmt, welche der Recheneinheit 16 durch eine Ausleseinheit 17 bereitgestellt werden. Die Recheneinheit 16 bestimmt die Reißlänge mittels eines Modells, welches eine Abhängigkeit der Reißlänge der Faserstoffbahn 11 von der Elastizität und von den Regelgrößen herstellt, wobei hierbei des weiteren im Labor 18 gemessene Werte der Reißlänge und Werte der Regelgrößen berücksichtigt werden, die bei der Herstellung der im Labor 18 gemessenen Proben eingestellt waren.

### Bezugszeichenliste

- 1: Maschine
- 2: Aufwickelvorrichtung
- 3: Faserstoffbahn
- 4: Walze
- 5: Bahnlaufrichtung (Pfeil)
- 6: Walze
- 7: Recheneinheit
- 10: Maschine
- 11: Faserstoffbahn
- 12: Walze
- 13: Walze
- 14: Walze
- 15: Bahnlaufrichtung (Pfeil)
- 16: Recheneinheit
- 17: Ausleseinheit
- 18: Labor

## Patentansprüche

1. Verfahren zur Bestimmung zumindest einer Dehnungseigenschaft einer in einer Maschine (1; 10) zur Herstellung einer Faserstoffbahn (3; 11) laufenden Bahn mit folgenden Schritten:
- Führen der Bahn über zumindest zwei aufeinander folgende angetriebene Walzen (4, 6; 12, 13, 14), wobei die Bahn zwischen aufeinander folgenden angetriebenen Walzen (4, 6; 12, 13, 14) jeweils zumindest abschnittweise ungestützt ist;
- Ermittlung der Zugkraft in der Bahn zwischen den aufeinander folgenden Walzen (4, 6; 12, 13, 14);
- Ermittlung der Differenzgeschwindigkeit der aufeinander folgenden Walzen (4, 6; 12, 13, 14) zueinander; und
- Bestimmung der zumindest einen Dehnungseigenschaft der zwischen den angetriebenen Walzen (4, 6; 12, 13, 14) geführten Bahn unter Berücksichtigung der zumindest einen Differenzgeschwindigkeit und der zumindest einen Zugkraft.

2. Verfahren nach Anspruch 1,
**dadurch gekennzeichnet,**
**dass** zur Bestimmung einer in Bahnlaufrichtung unterschiedlichen Dehnungseigenschaft der Bahn, die zeitliche Änderung der Zugkraft und/oder die zeitliche Änderung der Differenzgeschwindigkeit berücksichtigt werden.

3. Verfahren nach Anspruch 1,
**dadurch gekennzeichnet,**
**dass** zur Bestimmung einer in Bahnlaufrichtung unterschiedlichen Dehnungseigenschaft der Bahn, die zeitliche Änderung der jeweiligen Zugkraft ermittelt und die Differenzgeschwindigkeit zwischen den aufeinander folgenden Walzen (4, 6; 12, 13, 14) zeitlich konstant gehalten wird.

4. Verfahren nach Anspruch 1,
**dadurch gekennzeichnet,**
**dass** zur Bestimmung einer in Bahnlaufrichtung unterschiedlichen Dehnungseigenschaft der Bahn, die zeitliche Änderung der jeweiligen Differenzgeschwindigkeit ermittelt und die Zugkraft der Bahn zwischen den aufeinander folgenden Walzen (4, 6; 12, 13, 14) zeitlich konstant gehalten wird.

5. Verfahren nach einem der Ansprüche 1 bis 4,
**dadurch gekennzeichnet,**
**dass** die Bahn eine Faserstoffbahn (3; 11), insbesondere eine Papier-, Karton- oder Tissuebahn ist.

6. Verfahren nach einem der Ansprüche 1 bis 4,
**dadurch gekennzeichnet,**
**dass** die Bahn eine Bespannung, insbesondere ein Formiersieb, ein Pressfilz, ein Trockensieb oder ein glattes Transferband ist.

7. Verfahren nach einem der Ansprüche 1 bis 6,
**dadurch gekennzeichnet,**
**dass** die Zugkraft der Bahn unter Berücksichtigung der jeweiligen Antriebsdrehmomente der Walzenantriebe ermittelt wird.

8. Verfahren nach Anspruch 7,
**dadurch gekennzeichnet,**
**dass** die Antriebsdrehmomente aus der Kenntnis der Werte der Antriebsparameter der Walzenantriebe, insbesondere der elektrischen Spannung, dem elektrischen Strom oder der Leistungsaufnahme, ermittelt werden.

9. Verfahren nach einem der Ansprüche 7 oder 8,
**dadurch gekennzeichnet,**
**dass** zur Ermittlung der Zugkraft Kenndaten der Getriebe der Walzenantriebe und/oder Kenndaten der Walzen (4, 6; 12, 13, 14), wie insbesondere Reibwiderstand der Lagerung der Walze (4, 6; 12, 13, 14), berücksichtigt werden.

10. Verfahren nach einem der Ansprüche 7 bis 9,
**dadurch gekennzeichnet,**
**dass** bei der Bestimmung der zumindest einen Dehnungseigenschaft Reibwerte zwischen der Bahn und den angetriebenen Walzen (4, 6; 12, 13, 14) berücksichtigt werden.

11. Verfahren nach einem der Ansprüche 1 bis 10,
**dadurch gekennzeichnet,**
**dass** zwischen den angetriebenen Walzen (4, 6; 12, 13, 14) eine oder mehrere nicht angetriebene Walzen angeordnet sind, über welche die Bahn geführt wird.

12. Verfahren nach einem der Ansprüche 1 bis 11,
**dadurch gekennzeichnet,**
**dass** die zumindest eine Dehnungseigenschaft zwischen zwei aufeinander folgenden angetriebenen Walzen (4, 6; 12, 13, 14) bestimmt wird.

13. Verfahren nach Anspruch 12,
**dadurch gekennzeichnet,**
**dass** die zwei aufeinander folgenden angetriebenen Walzen (4, 6; 12, 13, 14) die beiden letzten Walzen (4, 6; 12, 13, 14) der Maschine (1; 10) zur Herstellung der Faserstoffbahn (3; 11) sind.

14. Verfahren nach Anspruch 13,
**dadurch gekennzeichnet,**
**dass** die letzte Walze die Walze (4) ist, auf welche die Bahn aufgewickelt wird.

15. Verfahren nach einem der Ansprüche 1 bis 11,
**dadurch gekennzeichnet,**
**dass** die zumindest eine Dehnungseigenschaft zwischen drei aufeinander folgenden angetriebenen Walzen (12, 13, 14)bestimmt wird.

16. Verfahren nach Anspruch 15,
**dadurch gekennzeichnet,**
**dass** die zumindest eine Dehnungseigenschaft aus der zeitlichen Änderung des Antriebsdrehmoments der mittleren Walze (13) der drei angetriebenen Walzen (12, 13, 14) bestimmt wird.

17. Verfahren nach Anspruch 15,
**dadurch gekennzeichnet,**
**dass** die zumindest eine Dehnungseigenschaft aus dem Integral des Antriebsdrehmoments der mittleren Walze (13) der drei angetriebenen Walzen (12, 13, 14) als Funktion der Zeit bestimmt wird.

18. Verfahren nach Anspruch 17,
**dadurch gekennzeichnet,**
**dass** über die Zeit integriert wird, die ein Ort auf der Bahn benötigt um von der ersten angetriebenen Walze (12) zur dritten angetriebenen Walze (14) zu gelangen.

19. Verfahren nach einem der Ansprüche 1 bis 18,
**dadurch gekennzeichnet,**
**dass** die Dehnungseigenschaft die Elastizität ist.

20. Verfahren nach einem der Ansprüche 1 bis 19,
**dadurch gekennzeichnet,**
**dass** aus der ermittelten Dehnungseigenschaft der Faserstoffbahn (3; 11) zumindest eine Festigkeitseigenschaft der Faserstoffbahn (3; 11) ermittelt wird.

21. Verfahren nach Anspruch 20,
**dadurch gekennzeichnet,**
**dass** zumindest eine die Festigkeitseigenschaft beeinflussenden Regelgröße bestimmt wird und mittels eines Modells, welches eine Abhängigkeit der Festigkeitseigenschaft von der Dehnungseigenschaft und von der Regelgröße herstellt, die Festigkeitseigenschaft ermittelt wird.

22. Verfahren nach Anspruch 20,
**dadurch gekennzeichnet,**
**dass** zumindest ein die Festigkeitseigenschaft beeinflussenden Parameter bestimmt wird und mittels eines Modells, welches eine Abhängigkeit der Festigkeitseigenschaft von der Dehnungseigenschaft und von dem Parameter herstellt, die Festigkeitseigenschaft ermittelt wird.

23. Verfahren nach Anspruch 21 oder 22,
**dadurch gekennzeichnet,**
**dass** das Modell zusätzlich die Sorte der Faserstoffbahn (3; 11) und/oder spezifische Eigenarten des verwendeten Herstellungsprozesses und/oder die Bauart der die Faserstoffbahn (3; 11) herstellenden Maschine (1; 10) berücksichtigt.

24. Verfahren nach Anspruch 21. 22 oder 23,
**dadurch gekennzeichnet,**
**dass** das Modell im Labor (18) gemessene Werte der zumindest einen Festigkeitseigenschaft berücksichtigt.

25. Verfahren nach einem der Ansprüche 20 bis 24,
**dadurch gekennzeichnet,**
**dass** die zumindest eine Festigkeitseigenschaft der Faserstoffbahn (3; 11) umfasst: deren Spaltbarkeit, deren Längs- und/oder Querbruchdehnbarkeit, deren Tensile, deren Reißlänge.
